# FASCICULE DE BREVET EUROPEEN

(11) **EP 1 421 931 B1**
(45) Date de publication et mention de la délivrance du brevet: **02.07.2008**
(21) Numéro de dépôt: 03292516.6
(22) Date de dépôt: 10.10.2003
(51) Int. Cl.: A61K 8/46, A61K 8/04, A61K 8/25, A61K 8/29, A61K 8/35, A61K 8/40, A61K 8/49, A61K 8/58, A61Q 17/04

(54) **Composition antisolaire vaporisable à base de microparticules sphériques de silice poreuse et dispositifs de pressurisation la contenant**
Versprühbare Sonnenschutzzubereitung auf der Basis von sphärischen Kieselsäure Mikropartikeln und Druckbehälter die Sonnenschutzzubereitung enthaltend
Vaporisable sunscreen composition on the basis of sperical silica microparticles and pressurized container comprising this composition

(30) Priorité: 21.11.2002 FR 0214599
(43) Date de publication de la demande: 26.05.2004
(73) Titulaire: L'ORÉAL, 75008 Paris (FR)
(72) Inventeur: Josso, Martin, 75007 Paris (FR)
(74) Mandataire: Miszputen, Laurent

(56) Documents cités:
- EP-A- 0 679 382
- EP-A- 0 968 703
- US-A- 5 939 079
- US-A- 6 004 567
- US-A1- 2001 036 466
- US-B1- 6 171 602
- US-B1- 6 258 857

## Description

La présente invention concerne un dispositif de pressurisation comprenant au moins (A) un réservoir contenant au moins une composition destinée à la protection de la peau et/ou des cheveux contre le rayonnement ultraviolet, caractérisée par le fait qu'elles se présente sous forme d'émulsion, simple ou complexe et qu'elle comprend, dans un support aqueux cosmétiquement acceptable au moins :
(a) un système photoprotecteur capable de filtrer le rayonnement UV ;
(b) des microparticules sphériques de silice poreuse
et (B) des moyens permettant de mettre sous pression ladite composition.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions anti-solaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV sur la dose de rayonnement UV nécessaire pour atteindre le seuil érythématogène sans filtre UV.

Aussi, il existe un besoin croissant de produits solaires ayant un indice de protection élevé. Les indices de protection élevés peuvent être atteints en incorporant plus de filtres à des concentration élevées. Ceci n'est pas toujours réalisable dans la mesure où l'addition de quantités importantes de filtres augmente considérablement ie coût des formulations solaires et les risques d'irritation de la peau.

Les produits solaires présentés sous forme de spray sont de plus en plus recherchés par les consommateurs, à cause de leur facilité d'utilisation et de leur agrément cosmétique.

A la différence des laits et des crèmes solaires classiques, il est particulièrement difficile d'obtenir des compositions solaires sous forme de spray ayant un indice de protection élevé.

On connaît dans le brevet US 6,258,857 des concentrés liquides destinés à être distribués au moyen d'un dispositif de pressurisation. Ces concentrés liquides se présentent généralement sous forme de dispersion aqueuse de particules de silice poreuse portant un actif tel qu'un insectifuge ou un filtre UV en association avec de fines particules de résine synthétique et/ou d'un polymère d'acide acrylique en présence d'un agent alcalin.

Les microparticules de silice poreuses sont connues comme charges dans la préparation de nombreuses formulations cosmétiques comme le décrit les documents US 2001/0036466, EP968703, EP679382, US 6,171,602, US5,939,079.

Or, à la suite d'importantes recherches menées dans le domaine de la photoprotection évoqué ci-dessus, la Demanderesse a découvert de manière surprenante que l'utilisation de microparticules sphériques de silice poreuse dans un dispositif de pressurisation contenant une composition telle que définie ci-dessous contenant au moins un système filtrant les radiations UV, il était possible d'obtenir une composition antisolaire ayant des indices de protection supérieurs à ceux qui peuvent être obtenus avec le même système photoprotecteur seul.

Cette découverte est à la base de la présente invention.

Ainsi, conformément à un premier objet de la présente invention, il est proposé un dispositif de pressurisation comprenant au moins (A) un réservoir contenant au moins une composition destinée à la protection de la peau et/ou des cheveux contre le rayonnement ultraviolet, caractérisée par le fait qu'elles caractérisé par le fait que ladite composition se présente sous forme d'émulsion, simple ou complexe et qu'elle comprend, dans un support aqueux cosmétiquement acceptable au moins :
(a) un système photoprotecteur capable de filtrer le rayonnement UV ;
(b) des microparticules sphériques de silice poreuse
et (B) des moyens permettant de mettre sous pression ladite composition.

Selon l'invention, on entend désigner de manière générale par système photoprotecteur capable de filtrer le rayonnement UV, tout composé ou toute association de composés qui, par des mécanismes connus en soi d'absorption et/ou de réflexion et/ou diffusion du rayonnement UV-A et/ou UV-B, permet d'empêcher, ou du moins limiter, la mise en contact dudit rayonnement avec une surface (peau, cheveux,) sur laquelle ce ou ces composés ont été appliqués. En d'autres termes, ces composés peuvent être des filtres organiques photoprotecteurs absorbeurs d'UV ou des (nano)pigments minéraux diffuseurs et/ou réflecteurs d'UV, ainsi que leurs mélanges.

Selon l'invention, on entend désigner de manière générale par "composition vaporisable", toute composition susceptible de produire sous pression dans un dispositif approprié de fines particules.

D'autres caractéristiques, aspects et avantages de la présente invention apparaîtront à la lecture de la description détaillée qui va suivre.

Les microparticules sphériques de silice poreuse conformes à l'invention ont de préférence une taille moyenne de particule allant de 0.5 à 20 µm et plus particulièrement de 3 à 15 µm.

Elles ont de préférence une surface spécifique allant de 50 à 1000 m²/g et plus particulièrement de 150 à 800 m²/g.

Elles ont de préférence un volume poreux spécifique allant de 0,5 à 5 ml/g et plus particulièrement de 1 à 2 ml/g

A titre d'exemple de microbilles de silice poreuse, on peut utiliser les produits commerciaux suivants :
Silica Beads SB 150 de Myoshi
Sunsphere H-51 de Asahi Glass
Sunsil 130 de Sunjin.
Spherica P-1500 de Ikeda Corporation
Sylosphere de Fuji Silysia

Les microparticules sphériques de silice poreuse conformes à la présente invention sont utilisées dans les compositions conformes à l'invention à des concentrations allant de préférence de 0,1 à 10% en poids par rapport au poids total de la composition et plus particulièrement de 0,2 à 5% en poids.

Selon l'invention, le système photoprotecteur peut être constitué par un ou plusieurs filtres organiques et/ou un ou plusieurs (nano)pigments minéraux.

Les filtres organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de (β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US 5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres organiques actifs dans l'UV-A et/ou l'UV-B, on peut citer désignés ci-dessus sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés du dibenzoylméthane :

Butyl Methoxydibenzoylmethane vendu notamment sous le nom commercial « PARSOL 1789 » par HOFFMANN LAROCHE,
Isopropyl Dibenzoylmethane,

### Dérivés cinnamiques :

Ethylhexyl Methoxycinnamate vendu notamment sous le nom commercial « PARSOL MCX » par HOFFMANN LAROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
   - Diisopropyl Methylcinnamate,
Glyceryl Ethylhexanoate Dimethoxycinnamate

### Dérivés de β,β-diphénylacrylate:

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12,
le 2-(4-diéthylamino-2-hydroxybenzoyl)-benzoate de n-hexyle

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
   - Terephthalylidene Dicamphor Sulfonic Acid fabriqué sous le nom « MEXORYL SX » par CHIMEX,
Polyacrylamidomethyl Benzylidene Camphor fabriqué sous le nom « MESORYL SW » par CHIMEX,

### Dérivés de benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés de triazine :

Anisotriazine vendu sous le nom commercial «TINOSORB S » par CIBA SPECIALTY CHEMICALS
Ethylhexyl triazone vendu notamment sous le nom commercial «UVINUL T150 » par BASF,
Diethylhexyl Butamido Triazone vendu sous le nom commercial « UVASORB HEB » par SIGMA 3V
la 2,4,6- tris-(4'amino-benzalmalonate de diisobutyle)-s-triazine.

### Dérivés de benzotriazole :

Drometrizole Trisiloxane vendu sous le nom « Silatrizole » par RHODIA CHIMIE , Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés de benzalmalonate :

Polyorganosiloxane à fonctions benzalmalonate vendu sous la dénomination commerciale « PARSOL SLX » par HOFFMANN LAROCHE

### Dérivés de 4,4-diarylbutadiène :

- 1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino]-6-(2-ethylhexyl)-imino-1, 3,5-triazine vendu sous le nom Uvasorb K2A par Sigma 3V ;
et leurs mélanges.

Les filtres organiques plus particulièrement préférés sont choisis parmi les composés suivants :
Ethylhexyl Salicylate,
Ethylhexyl Methoxycinnamate
Octocrylene,
Phenylbenzimidazole Sulfonic Acid,
Benzophenone-3,
Benzophenone-4,
Benzophenone-5,
le 2-(4-diéthylamino-2-hydroxybenzoyl)-benzoate de n-hexyle
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine et leurs mélanges.

Les filtres inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Le système photoprotecteur selon l'invention est généralement présent dans les compositions selon l'invention à une teneur allant de 0,1 % à 30 % en poids et de préférence de 0,5 à 15 % , en poids, par rapport au poids total de la composition.

Les compositions vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les compositions selon l'invention peuvent également contenir des agents de bronzage et/ou de brunissage artificiels de la peau (agents autobronzants).

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

Les agents autobronzants mono ou polycarbonylés sont généralement présents dans les compositions selon l'invention dans des proportions allant de 0,1 à 10% en poids par rapport au poids total de la composition, et de préférence de 0,2 à 8% en poids par rapport au poids total de la composition

Les compositions de l'invention peuvent comprendre en outre des adjuvants cosmétiques classiques notamment choisis parmi les corps gras, les solvants organiques, les épaississants, les adoucissants, les opacifiants, les stabilisants, les émollients, les agents anti-mousse, les agents hydratants, les parfums, les conservateurs, les polymères, les charges, les séquestrants, les bactéricides et/ou les absorbeurs d'odeur. les agents alcalinisants ou acidifiants, les tensio-actifs, les émulsionnants, les anti radicaux libres, les antioxydants, les vitamines comme les vitamines E et C, les α-hydroxyacides ou tout autre ingrédient habituellement utilisé en cosmétique, en particulier pour la fabrication de compositions antisolaires aqueuses vaporisables.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les polymères acryliques réticulés comme les Carbomer, les polymères réticulés acrylates/C10-C30alkylacrylates du type Pemulen ou le polyacrylate-3 vendu sous le nom VISCOPHOBE DB 1000 par Amerchol ; les polyacrylamides tels que l'émulsion polyacrylamide, C13-C14 isoparraffine et laureth-7 vendue sous le nom SEPIGEL 305 par SEPPIC, les homopolymères ou copolymères d'AMPS tel l'HOSTACERIN AMPS vendu par CLARIANT, les gommes de guar et celluloses modifiées ou non telles que la gomme de guar hydroxypropylée, la méthylhydroxyéthylcellulose et l'hydroxypropylméthyl cellulose, la gomme de xanthane, les silices nanométriques de type Aerosil.

Les agents émulsionnants ou stabilisants d'émulsions peuvent être choisis parmi les tensioactifs non ioniques, anioniques ou cationiques. Parmi les stabilisants d'émulsion, on utilisera plus particulièrement les polymères d'acide isophtalique ou d'acide sulfoisophtalique, et en particulier les copolymères de phtalate / sulfoisophtalate / glycol (par exemple diéthylèneglycol / Phtalate / isophtalate / 1,4-cyclohexane-diméthanol) vendus sous les dénominations "Eastman AQ polymer" (AQ35S, AQ38S, AQ55S, AQ48 Ultra) par la société Eastman Chemical.

Bien entendu, l'homme de l'art veillera à choisir le ou les éventuels composés complémentaires cités ci-dessus et/ou leurs quantités de manière telle que les propriétés avantageuses attachées intrinsèquement à l'association [système photoprotecteur + microbilles de silice poreuse] conforme à l'invention ne soit pas, ou substantiellement pas, altérée par la ou les adjonctions envisagées.

Les compositions concernées par l'invention peuvent être préparées selon les techniques bien connues de l'homme de l'art, destinées à la préparation des formulations vaporisables.

Les compositions selon l'invention se présentent de préférence sous forme d'émulsion, simple ou complexe (H/E, E/H, H/E/H ou E/H/E) telle qu'une crème, un lait et plus particulièrement sous la forme d'une lotion.

Plus préférentiellement, les compositions selon l'invention se présentent sous la forme d'une émulsion huile-dans-eau ou eau-dans-huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Des exemples concrets, mais nullement limitatifs, illustrant l'invention, vont maintenant être donnés.

Dans tous les exemples qui suivent, les quantités sont exprimées en % de poids par rapport au poids total de la composition.

### EXEMPLE 1 :

On a préparé une formulation antisolaire A selon l'invention susceptible d'être conditionnée dans un spray non-aérosol ou aérosol et d'être distribuée sous forme de fines particules.

| **Formulation A** | **Quantité % en poids** |
|---|---|
| Octocrylene (UVINUL N539) | 10 |
| Ethylhexyl triazone (UVINUL T150) | 1 |
| Drometrizole trisiloxane (MEXORYL XL) | 3 |
| Butyl methoxydibenzoylméthane (Parsol 1789) | 3 |
| Terephtalylidene dicamphor sulfonic acid (MEXORYL SX) | 0.5 |
| Titanium dioxide | 5 |
| C₁₂-C₁₅ alkyl benzoate | 6 |
| Huile de jojoba | 1 |
| Beurre de Karité | 1 |
| Cyclohexasiloxane (DC Fluid 246 de Dow Corning) | 5 |
| Glycérine | 6 |
| Propylèneglycol | 6 |
| Microbilles de silice poreuse (Silica Beads SB 150 de Myoshi) | 1 |
| Copolymère de Diglycol/Cyclohexanedimethanol/isophtalates/Sulfoisophtal ates (AQ 38S de EASTMAN) | 1 |
| Polyacrylate-3 en émulsion à 25% (VISCOPHOBE DB 1000 de Amerchol) | 0.5 |
| Mélange de tocophérols naturels et huile de soja | 0.2 |
| Triéthanolamine | qs |
| Conservateurs | qs |
| Eau | qsp 100 |

On a ensuite préparé une formulation antisolaire B comparative, de même support que formulation A mais ne contenant pas de microbilles de silice poreuse.

Pour chacune des compositions A et B, on a ensuite déterminé le facteur de protection solaire (SPF) qui lui était attaché. Celui-ci a été déterminé en utilisant la méthode in vitro décrite par B.L. DIFFEY et al. dans J. Soc. Cosmet. Chem. 40-127-133 (1989) ; cette méthode consiste à déterminer les facteurs de protection monochromatiques dans une gamme de longueurs d'onde de 290 à 400 nm et à calculer à partir de ceux-ci le facteur de protection solaire selon une équation mathématique donnée. La mesure a été réalisée avec un pas de 1 nm sur un appareil UV-1000S de la société Labsphere, 2 mg/cm2.de produit étant étalé sur ruban Transpore®.

Les résultats (valeur moyenne correspondant à cinq essais) sont regroupés dans le tableau (I) ci-dessous :

**Tableau (I) :**

| Composition | A (invention) avec microbilles de silice poreuse | B (hors invention) sans microbille de silice poreuse |
|---|---|---|
| SPF moyen (écart type) | 21.5 (2.6) | 15.5 (2.8) |

Ces résultats montrent clairement que l'ajout dans un support vaporisable de microparticules sphériques de silice poreuse à un système photoprotecteur constitué d'octocrylene, de Butyl methoxydibenzoylméthane, d'éthylhexyl triazone, de Drometrizole trisiloxane, de Terephtalylidene dicamphor sulfonic acid et de nanopigments de TiO₂, permet d'augmenter significativement son facteur de protection solaire.

## Revendications

1. Dispositif comprenant au moins (A) un réservoir contenant au moins une composition destinée à la protection de la peau et/ou des cheveux contre le rayonnement ultraviolet et (B) des moyens permettant de mettre sous pression ladite composition, **caractérisé par le fait que** ladite composition se présente sous forme d'émulsion, simple ou complexe et comprend, dans un support aqueux cosmétiquement acceptable au moins :
(a) un système photoprotecteur capable de filtrer le rayonnement UV ;
(b) des microparticules sphériques de silice poreuse.

2. Dispositif selon la revendication 1, où les microparticules sphériques de silice poreuse ont une taille moyenne de particule allant de 0.5 à 20 µm et plus particulièrement de 3 à 15 µm.

3. Dispositif selon la revendication 1 ou 2, où les microparticules sphériques de silice poreuse ont une surface spécifique allant de 50 à 1000 m²/g et plus particulièrement de 150 à 800 m²/g.

4. Dispositif selon la revendication 1 à 3, où les microparticules sphériques de silice poreuse ont un volume poreux spécifique allant de 0,5 à 5 ml/g et plus particulièrement de 1 à 2 ml/g.

5. Dispositif selon l'une quelconque des revendications 1 à 4, où les microparticules sphériques de silice poreuse sont présentes dans la composition à des concentrations allant de préférence de 0,1 à 10% en poids par rapport au poids total de la composition et plus particulièrement de 0,2 à 5% en poids.

6. Dispositif selon l'une quelconque des revendications 1 à 5, où le système photoprotecteur est constitué par un ou plusieurs filtres organiques et/ou un ou plusieurs (nano)pigments minéraux.

7. Dispositif selon la revendication 6, où les filtres organiques sont choisis parmi les anthranilates ; les dérivés cinnamiques ; les dérivés de dibenzoylméthane ; les dérivés salicyliques, les dérivés du camphre ; les dérivés de triazine; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes et leurs mélanges.

8. Dispositif selon la revendication 7, où les filtres organiques sont choisis parmi
Ethylhexyl Salicylate,
Ethylhexyl Methoxycinnamate
Octocrylene,
Phenylbenzimidazole Sulfonic Acid,
Benzophenone-3,
Benzophenone-4,
Benzophenone-5,
2-(4-diéthylamino-2-hydroxybenzoyl)-benzoate de n-hexyle
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-iminoJ-6-(2-ethylhexyl)-imino-1;3,5-triazine et leurs mélanges.

9. Dispositif selon la revendication 6, où les pigments ou les nanopigments sont choisis parmi les oxydes métalliques enrobés ou non.

10. Dispositif selon la revendication 9, où le ou les agents filtrant les radiations UV minéraux sont choisis parmi les nanopigments d'oxyde de titane, de fer, de zinc, de zirconium ou de cérium, enrobés ou non.

11. Dispositif selon l'une quelconque des revendications 1 à 10, où le système photoprotecteur est présent à une teneur allant de 0,1 % à 30 % en poids et de préférence de 0,5 à 15 %, en poids, par rapport au poids total de la composition.

12. Dispositif selon l'une quelconque des revendications 1 à 11, **caractérisé par le fait que** la composition comprend en plus au moins un agent propulseur.

13. Dispositif selon la revendication 12, où le ou les agents propulseurs sont présents dans des quantités allant de 15 à 50% en poids par rapport au poids total de la composition.

14. Dispositif selon l'une quelconque des revendications 1 à 13, **caractérisée par le fait que** la composition contient en plus au moins un agent autobronzant.

15. Dispositif selon la revendication 14, où le ou les agents autobronzants sont choisis parmi les composés mono ou polycarbonylés.

16. Dispositif selon la revendication 15, où le ou les agents autobronzants sont choisis parmi l'isatine, l'alloxane, la ninhydrine, le glycéraldéhyde, l'aldéhyde mésotartrique, la glutaraldéhyde, l'érythrulose, les dérivés de pyrazolin-4,5-diones, la dihydroxyacétone (DHA), les dérivés de 4,4-dihydroxypyrazolin-5-ones.

17. Dispositif selon l'une quelconque des revendications 14 à 16, où l'agent autobronzant est la dihydroxyacétone.

18. Composition selon l'une quelconque des revendications 1 à 17, où l'agent ou les agents autobronzants sont présents dans des proportions allant de 0,1 à 10% en poids par rapport au poids total de la composition, et de préférence de 0,2 à 8% en poids par rapport au poids total de la composition.

19. Dispositif selon l'une quelconque des revendications 1 à 17, où la composition contient en outre au moins un adjuvant cosmétique choisi parmi les corps gras, les solvants organiques, les épaississants, les adoucissants, les opacifiants, les stabilisants, les émollients, les agents anti-mousse, les agents hydratants, les parfums, les conservateurs, les polymères, les charges, les séquestrants, les bactéricides et/ou les absorbeurs d'odeur, les agents alcalinisants ou acidifiants, les tensio-actifs, les émulsionnants, les anti radicaux libres, les antioxydants, les vitamines comme les vitamines E et C, les α-hydroxyacides.

20. Dispositif selon l'une quelconque des revendications 1 à 19, où la composition contient en outre au moins un polymère d'acide isophtalique ou d'acide sulfoisophtalique.

21. Dispositif selon la revendication 19, où ledit polymère d'acide isophtalique ou d'acide sulfoisophtalique est un copolymère de phtalate / sulfoisophtalate / glycol et plus particulièrement un copolymère de diéthylèneglycol / Phtalate / isophtalate / 1,4-cyclohexane-diméthanol.

22. Dispositif selon la revendication 21, **caractérisée par** le fait **caractérisé par le fait que** la composition se présente sous la forme d'une émulsion huile-dans-eau ou eau-dans-huile.

23. Dispositif selon l'une quelconque des revendications 1 à **22, caractérisé par le fait qu'**il s'agit d'une pompe non-aérosol.

24. Dispositif selon l'une quelconque des revendications 1 à **22, caractérisé par le fait qu'**il s'agit d'un récipient aérosol ou d'une pompe aérosol.

## Claims

1. Device comprising at least (A) a reservoir containing at least one composition intended for protecting the skin and/or the hair against ultraviolet radiation, and (B) means which make it possible to put the said composition under pressure, **characterized in that** the said composition is provided in the form of a simple or complex emulsion and comprises, in a cosmetically acceptable aqueous carrier, at least:
(a) a photoprotective system capable of screening out UV radiation;
(b) spherical microparticles of porous silica.

2. Device according to Claim 1, where the spherical microparticles of porous silica have a mean particle size ranging from 0.5 to 20 µm and more particularly from 3 to 15 µm.

3. Device according to Claim 1 or 2, where the spherical microparticles of porous silica have a specific surface ranging from 50 to 1 000 m²/g and more particularly from 150 to 800 m²/g.

4. Device according to Claim 1 to 3, where the spherical microparticles of porous silica have a specific pore volume ranging from 0.5 to 5 ml/g and more particularly from 1 to 2 ml/g.

5. Device according to any one of Claims 1 to 4, where the spherical microparticles of porous silica are present in the composition at concentrations preferably ranging from 0.1 to 10% by weight relative to the total weight of the composition and more particularly from 0.2 to 5% by weight.

6. Device according to any one of Claims 1 to 5, where the photoprotective system consists of one or more organic screening agents and/or one or more inorganic (nano)pigments.

7. Device according to Claim 6, where the organic screening agents are chosen from anthranilates; cinnamic derivatives; dibenzoylmethane derivatives; salicylic derivatives, camphor derivatives; triazine derivatives; benzophenone derivatives; β,β'-diphenyl acrylate derivatives; benzotriazole derivatives; benzalmalonate derivatives; benzimidazole derivatives; imidazolines; bisbenzoazolyl derivatives; p-aminobenzoic acid (PABA) derivatives; methylenebis(hydroxyphenylbenzotriazole) derivatives; benzoxazole derivatives; screening polymers and screening silicones; dimers derived from α-alkylstyrene; 4,4-diarylbutadiene derivatives and mixtures thereof.

8. Device according to Claim 7, where the organic screening agents are chosen from
Ethylhexyl Salicylate,
Ethylhexyl Methoxycinnamate,
Octocrylene,
Phenylbenzimidazole Sulphonic Acid,
Benzophenone-3,
Benzophenone-4,
Benzophenone-5,
n-Hexyl 2-(4-diethylamino-2-hydroxybenzoyl)benzoate,
4-Methylbenzylidene camphor,
Terephthalylidene Dicamphor Sulphonic Acid,
Disodium Phenyl Dibenzimidazole Tetra-sulphonate,
2,4,6-Tris(diisobutyl 4'-aminobenzalmalonate)-s-triazine,
Anisotriazine,
Ethylhexyl triazone,
Diethylhexyl Butamido Triazone,
Methylene bis-Benzotriazolyl
Tetramethylbutylphenol,
Drometrizole Trisiloxane,
Polysilicone-15
1,1-Dicarboxy-(2,2'-dimethylpropyl)-4,4-diphenyl-butadiene,
2,4-bis[5-1(dimethylpropyl)benzoxazol-2-yl(4-phenyl)imino]-6-(2-ethylhexyl)imino-1,3,5-triazine,
and mixtures thereof.

9. Device according to Claim 6, where the pigments or nanopigments are chosen from metal oxides, coated or otherwise.

10. Device according to Claim 9, where the inorganic agent(s) screening out UV radiation are chosen from nanopigments of titanium oxide, iron oxide, zinc oxide, zirconium oxide or cerium oxide, coated or otherwise.

11. Device according to any one of Claims 1 to 10, where the photoprotective system is present in an amount ranging from 0.1% to 30% by weight and preferably from 0.5 to 15% by weight, relative to the total weight of the composition.

12. Device according to any one of Claims 1 to 11, **characterized in that** the composition furthermore comprises at least one propelling agent.

13. Device according to Claim 12, where the propelling agent(s) is (are) present in quantities ranging from 15 to 50% by weight relative to the total weight of the composition.

14. Device according to any one of Claims 1 to 13, **characterized in that** the composition furthermore contains at least one self-tanning agent.

15. Device according to Claim 14, where the self-tanning agent(s) is (are) chosen from mono- or polycarbonyl compounds.

16. Device according to Claim 15, where the self-tanning agent(s) is (are) chosen from isatin, alloxan, ninhydrin, glyceraldehyde, mesotartaric aldehyde, glutaraldehyde, erythrulose, pyrazolin-4,5-dione derivatives, dihydroxyacetone (DHA), 4,4-dihydroxypyrazolin-5-one derivatives.

17. Device according to any one of Claims 14 to 16, where the self-tanning agent is dihydroxyacetone.

18. Device according to any one of Claims 1 to 17, where the self-tanning agent(s) is (are) present in proportions ranging from 0.1 to 10% by weight relative to the total weight of the composition, and preferably from 0.2 to 8% by weight relative to the total weight of the composition.

19. Device according to any one of Claims 1 to 17, where the composition additionally contains at least one cosmetic adjuvant chosen from fatty substances, organic solvents, thickeners, demulcents, opacifiers, stabilizers, emollients, anti-foaming agents, moisturizing agents, perfumes, preservatives, polymers, fillers, sequestrants, bactericides and/or odour absorbers, alkalinizing or acidifying agents, surfactants, emulsifiers, anti-free radical agents, antioxidants, vitamins such as vitamins E and C, α-hydroxy acids.

20. Device according to any one of Claims 1 to 19, where the composition additionally contains at least one polymer of isophthalic acid or of sulphoisophthalic acid.

21. Device according to Claim 19, where the said polymer of isophthalic acid or of sulphoisophthalic acid is a copolymer of phthalate/sulphoisophthalate/glycol and more particularly a copolymer of diethylene glycol/phthalate/isophthalate/1,4-cyclohexanedimethanol.

22. Device according to Claim 21, **characterized in that** the composition is provided in the form of an oil-in-water or water-in-oil emulsion.

23. Device according to any one of Claims 1 to 22, **characterized in that** it is a nonaerosol pump.

24. Device according to any one of Claims 1 to 22, **characterized in that** it is an aerosol container or an aerosol pump.

## Patentansprüche

1. Vorrichtung, die zumindest (A) ein Reservoir, das mindestens eine Zusammensetzung enthält, die für den Schutz der Haut und/oder der Haare gegen UV-Strahlung vorgesehen ist, und (B) Mittel umfasst, mit denen die Zusammensetzung unter Druck gesetzt werden kann, **dadurch gekennzeichnet, dass** die Zusammensetzung in Form einer einfachen oder komplexen Emulsion vorliegt und in einem kosmetisch akzeptablen, wässrigen Träger zumindest enthält:
(a) ein Lichtschutzsystem, das befähigt ist, die UV-Strahlung zu filtern;
(b) sphärische Mikropartikel von poröser Kieselsäure.

2. Vorrichtung nach Anspruch 1, wobei die sphärischen Mikropartikel von poröser Kieselsäure eine mittlere Partikelgröße von 0,5 bis 20 µm und insbesondere 3 bis 15 µm aufweisen.

3. Vorrichtung nach Anspruch 1 oder 2, wobei die sphärischen Mikropartikel von poröser Kieselsäure eine spezifische Oberfläche von 50 bis 1 000 m²/g, und insbesondere 150 bis 800 m²/g aufweisen.

4. Vorrichtung nach Anspruch 1 bis 3, wobei die sphärischen Mikropartikel von poröser Kieselsäure ein spezifisches Porenvolumen von 0,5 bis 5 ml/g und insbesondere 1 bis 2 ml/g aufweisen.

5. Vorrichtung nach einem der Ansprüche 1 bis 4, wobei die sphärischen Mikropartikel von poröser Kieselsäure in der Zusammensetzung in Konzentrationen von vorzugsweise 0,1 bis 10 Gew.-%, bezogen auf das Gesamtgewicht der Zusammensetzung, und insbesondere 0,2 bis 5 Gew.-% enthalten sind.

6. Vorrichtung nach einem der Ansprüche 1 bis 5, wobei das Lichtschutzsystem aus einem oder mehreren organischen Filtern und/oder einem oder mehreren anorganischen (Nano)pigmenten besteht.

7. Vorrichtung nach Anspruch 6, wobei die organischen Filter unter Anthranilaten; Zimtsäurederivaten; Dibenzoylmethanderivaten; Salicylsäurederivaten, Campherderivaten; Triazinderivaten; Benzophenonderivaten; β, β-Diphenylacrylatderivaten; Benzotriazolderivaten; Benzalmalonatderivaten; Benzimidazolderivaten; Imidazolinen; Bis-benzoazolylderivaten; Derivaten von p-Aminobenzoesäure (PABA); Methylen-bis(hydroxyphenylbenzotriazolderivaten); Benzoxazolderivaten; Filterpolymeren und Siliconfiltern; von α-Alkylstyrol abgeleiteten Dimeren; 4,4-Diarylbutadienen und deren Gemischen ausgewählt sind.

8. Vorrichtung nach Anspruch 7, wobei die organischen Filter ausgewählt sind unter
Ethylhexyl Salicylate
Ethylhexyl Methoxycinnamate
Octocrylene,
Phenylbenzimidazole Sulfonic Acid,
Benzophenone-3,
Benzophenone-4,
Benzophenone-5,
n-Hexyl-2-(4-diethylamino-2-hydroxybenzoyl)-benzoat,
4-Methylbenzylidene Camphor,
Terephthalylidene Dicamphor Sulfonic Acid,
Disodium Phenyl Dibenzimidazole Tetra-sulfonate,
2,4,6-Tris(diisobutyl-4'-aminobenzalmalonat)-s-triazin,
Anisotriazine,
Ethylhexyl Triazone,
Diethylhexyl Butamido Triazone,
Methylene bis-Benzotriazolyl Tetramethylbutylphenol,
Drometrizole Trisiloxane,
Polysilicone-15,
1,1-Dicarboxy-(2,2'-dimethylpropyl)-4,4-diphenylbutadien,
2,4-Bis[5-1(dimethylpropyl)benzoxazol-2-yl-(4-phenyl)-imino]-6-(2-ethylhexyl)-imino-1,3,5-triazin und deren Gemischen.

9. Vorrichtung nach Anspruch 6, wobei die Pigmente oder Nanopigmente unter den umhüllten oder nichtumhüllten Metalloxiden ausgewählt sind.

10. Vorrichtung nach Anspruch 9, wobei der oder die anorganischen Stoffe, die die UV-Strahlung filtern, unter den Nanopigmenten von Titanoxid, Eisenoxid, Zinkoxid, Zirconiumoxid oder Ceroxid ausgewählt sind, die umhüllt oder nichtumhüllt sind.

11. Vorrichtung nach einem der Ansprüche 1 bis 10, wobei das Lichtschutzsystem in einer Menge von 0,1 bis 30 Gew.-% und vorzugsweise 0,5 bis 15 Gew.-%, bezogen auf das Gesamtgewicht der Zusammensetzung, enthalten ist.

12. Vorrichtung nach einem der Ansprüche 1 bis 11, **dadurch gekennzeichnet, dass** die Zusammensetzung ferner mindestens ein Treibmittel enthält.

13. Vorrichtung nach Anspruch 12, wobei das oder die Treibmittel in Mengen von 15 bis 50 Gew.-%, bezogen auf das Gesamtgewicht der Zusammensetzung, enthalten sind.

14. Vorrichtung nach einem der Ansprüche 1 bis 13, **dadurch gekennzeichnet, dass** die Zusammensetzung ferner mindestens ein Selbstbräunungsmittel enthält.

15. Vorrichtung nach Anspruch 14, wobei das oder die Selbstbräunungsmittel unter den Verbindungen mit einer oder mehreren Carbonylfunktionen ausgewählt sind.

16. Vorrichtung nach Anspruch 15, wobei das oder die Selbstbräunungsmittel unter Isatin, Alloxan, Ninhydrin, Glyceraldehyd, Mesoweinsäurealdehyd, Glutaraldehyd, Erythrulose, Pyrazolin-4,5-dionderivaten, Dihydroxyaceton (DHA) und 4,4-Dihydroxypyrazolin-5-on-derivaten ausgewählt sind.

17. Vorrichtung nach einem der Ansprüche 14 bis 16, wobei es sich bei dem Selbstbräunungsmittel um das Dihydroxyaceton handelt.

18. Zusammensetzung nach einem der Ansprüche 1 bis 17, wobei das oder die Selbstbräunungsmittel in Mengenanteilen von 0,1 bis 10 Gew.-%, bezogen auf das Gesamtgewicht der Zusammensetzung, und vorzugsweise 0,2 bis 8 Gew.-%, bezogen auf das Gesamtgewicht der Zusammensetzung, enthalten sind.

19. Vorrichtung nach einem der Ansprüche 1 bis 17, wobei die Zusammensetzung ferner mindestens einen kosmetischen Zusatzstoff enthält, der unter den Fettsubstanzen, organischen Lösungsmitteln, Verdickungsmitteln, beruhigenden Stoffen, Trübungsmitteln, Stabilisatoren, Emollientien, Schaumverhütungsmitteln, Hydratisierungsmitteln, Parfums, Konservierungsmitteln, Polymeren, Füllstoffen, Maskierungsmitteln, Bakteriziden und/oder Geruchabsorbern, Alkalisierungsmitteln oder Ansäuerungsmitteln, grenzflächenaktiven Stoffen, Emulgatoren, Radikalfängern für freie Radikale, Antioxidantien, Vitaminen, wie Vitamin E und C, und α-Hydroxysäuren ausgewählt ist.

20. Vorrichtung nach einem der Ansprüche 1 bis 19, wobei die Zusammensetzung ferner mindestens ein Polymer von Isophthalsäure oder Sulfoisophthalsäure enthält.

21. Vorrichtung nach Anspruch 19, wobei das Polymer von Isophthalsäure oder Sulfoisophthalsäure ein Copolymer Phthalat/Isophthalat/Glycol und insbesondere ein Copolymer Diethylenglycol/Phthalat/Isophthalat/ 1,4-Cyclohexandimethanol ist.

22. Vorrichtung nach Anspruch 21, **dadurch gekennzeichnet, dass** die Zusammensetzung als Öl-in-Wasser-Emulsion oder Wasserin-Öl-Emulsion vorliegt.

23. Vorrichtung nach einem der Ansprüche 1 bis 22, **dadurch gekennzeichnet, dass** es sich um einen Nichtaerosolpumpflakon handelt.

24. Vorrichtung nach einem der Ansprüche 1 bis 22, **dadurch gekennzeichnet, dass** es sich um einen Aerosolbehälter oder Aerosolpumpflakon handelt.
